# EUROPEAN PATENT APPLICATION

(11) **EP 2 989 960 A1**
(43) Date of publication of application: **02.03.2016**
(21) Application number: 14873687.9
(22) Date of filing: 19.09.2014
(51) Int. Cl.: A61B 1/00, G02B 23/24

(54) **ENDOSCOPE SHEATH**

(30) Priority: 24.12.2013 JP 2013265324
(71) Applicant: Olympus Corporation, Shibuya-ku Tokyo 151-0072 (JP)
(72) Inventor: KANEKO Hiroyuki, Tokyo 151-0072 (JP)
(74) Representative: Schicker, Silvia
(86) International application number: PCT/JP2014/074906
(87) International publication number: WO 2015/098210

(57) **Abstract**

A sheath for an endoscope is provided that includes: a first tubular member having a longitudinal axis direction through-hole in which an insertion portion of an endoscope can be disposed in an insertable/extractable manner; a stopper member that has a holding hole that holds the first tubular member so as to be capable of advancing and retreating, and a concave portion forming an opening in a direction perpendicular to an axis of the holding hole; a contact member that is fixed to one end portion side of the first tubular member which is held so as to be capable of advancing and retreating inside the holding hole of the stopper member and which protrudes from one end face side of the stopper member, and that has a contact face that contacts against one end face of the stopper member; a second tubular member that includes a longitudinal axis direction through-hole in which the contact member and the stopper member can be slidably disposed, a locking portion that can be locked to an operation portion provided on a one end-face opening side of the longitudinal axis direction through-hole, a movement range setting hole that is a long hole leading to the longitudinal axis direction through-hole and that sets a longitudinal axis direction movement range of the contact member with which the first tubular member is integrated, and a positioning hole that is a through-hole leading to the longitudinal axis direction through-hole and that is formed so as to be alignable with respect to the concave portion of the stopper member that is provided on the other end-face opening side of the longitudinal axis direction through-hole; a positioning and fixing member that is disposed in the concave portion of the stopper member through the positioning hole of the second tubular member, and that positions and fixes the stopper member with respect to the second tubular member; and a knob portion that is fixed to the contact member through the movement range setting hole that the second tubular member includes.

## Description

### Technical Field

The present invention relates to a rigid tubular sheath for an endoscope, through which an insertion portion of a flexible endoscope is to be inserted.

### Background Art

When using an endoscope, a procedure is sometimes performed in which an elongated insertion portion of the endoscope is guided into a kidney through a urinary duct to perform observation or treatment. Among nephroscopes that are endoscopes for performing an observation or the like inside a kidney, there are nephroscopes in which an insertion portion is constructed to be rigid, and there are nephroscopes in which an insertion portion that is provided with a bending portion and a flexible tube portion is constructed to be flexible.

In an endoscope in which the insertion portion is rigid, after the insertion portion is inserted into the urinary duct, the insertion portion can be smoothly guided into a kidney. However, in a rigid endoscope, the observation range is restricted to the front in the insertion direction.

In contrast, in an endoscope in which the insertion portion is flexible, there is the problem that it is difficult to guide the insertion portion from the urinary duct into a kidney, and it takes time to master the technique for doing so. However, in the case of a flexible endoscope, the inside of the kidney can be observed over a wide range by causing the bending portion to perform a bending operation.

In recent years a nephroscope is available in which a bending portion is configured to be capable of bending 180 degrees in the upward direction and capable of bending 275 degrees in the downward direction. According to this nephroscope, it is possible to access the upper renal calyx and middle renal calyx by using the function for bending 180 degrees in the upward direction, and it is possible to access the lower renal calyx by using the function for bending 275 degrees in the downward direction.

A sheath for an endoscope apparatus is disclosed in Japanese Patent Application Laid-Open Publication No. 2010-253150. The aforementioned sheath for an endoscope apparatus includes a connection adapter that is engageable with and disengageable from an operation portion at a proximal end of a sheath portion that is fitted onto an insertion tube portion of the endoscope.

The sheath portion is constituted by connecting and fixing a flexible tube portion to a distal end of a rigid pipe portion. The sheath portion is configured so that an externally threaded tube portion provided at a proximal end of the sheath portion can be freely threaded onto or separated from a sheath attachment cylinder. The sheath attachment cylinder is configured to be movable within only a fixed range in an axial direction with respect to the connection adapter portion.

According to this configuration, by performing an operation that moves the sheath portion in the axial direction relative to the connection adapter portion, a state in which a distal end face of the sheath portion is positioned forward from a distal end face of the insertion tube portion, and a state in which the distal end face of the sheath portion is positioned rearward from the distal end face of the insertion tube portion can be entered, respectively.

The sheath portion of the sheath for an endoscope apparatus that is described above is made by connecting and fixing a rigid pipe portion and a flexible tube portion. Consequently, when the flexible tube portion is inserted into the urinary duct and guided into a kidney, the same problem arises as in the case of an endoscope having a flexible insertion portion. However, this problem can be solved by using only a rigid pipe portion to make the sheath portion.

However, there are individual differences between the lengths of insertion portions of endoscopes that are used in combination with the sheath for an endoscope apparatus disclosed in Japanese Patent Application Laid-Open Publication No. 2010-253150. Consequently, there is a risk that the situations described in the following items 1 to 3 will arise depending on the combination of the insertion portion and sheath portion.
1. The distal end face of the sheath portion will be disposed partway along or at a proximal end of a bending portion provided in the insertion portion.
2. Even in a case where the sheath portion is moved in the axial direction relative to the connection adapter portion, the distal end face of the sheath portion will continue to be located to the front of the distal end face of the insertion portion.
3. The bending portion of the insertion portion will not be exposed in a desired state from the distal end face of the sheath portion.

The present invention has been made in consideration of the above described circumstances, and an object of the present invention is to provide a sheath for an endoscope that, while absorbing an individual difference of a flexible insertion portion that is provided in an endoscope, enables smooth introduction of the insertion portion into an observation site, as well as observation over a wide area inside the observation site after introduction of the insertion portion.

### Disclosure of Invention

### Means for Solving the Problem

A sheath for an endoscope according to one aspect of the present invention includes: a first tubular member having a longitudinal axis direction through-hole in which an insertion portion of an endoscope having a distal end portion, a bending portion and a flexible tube portion can be disposed in an insertable/extractable manner; a stopper member that has a holding hole that holds the first tubular member so that the first tubular member is capable of advancing and retreating, and a concave portion forming an opening in a direction perpendicular to an axis of the holding hole; a contact member that is integrally fixed to one end portion side of the first tubular member which is held so as to be capable of advancing and retreating inside the holding hole of the stopper member and which protrudes from one end face side of the stopper member, and that has a contact face that contacts against one end face of the stopper member; a second tubular member that includes a longitudinal axis direction through-hole in which the contact member and the stopper member can be slidably disposed, a locking portion that is provided on a one end-face opening side of the longitudinal axis direction through-hole and that can be locked to an operation portion of the endoscope, a movement range setting hole that is a long hole leading to the longitudinal axis direction through-hole and that sets a longitudinal axis direction movement range of the contact member with which the first tubular member is integrated, and a positioning hole that is a through-hole leading to the longitudinal axis direction through-hole and that is formed so as to be alignable with respect to the concave portion of the stopper member that is provided on the other end-face opening side of the longitudinal axis direction through-hole; a positioning and fixing member that is disposed in the concave portion of the stopper member through the positioning hole that the second tubular member includes, and that positions and fixes the stopper member with respect to the second tubular member; and a knob portion that is threadedly disposed in the contact member through the movement range setting hole that the second tubular member includes.

### Brief Description of the Drawings

Fig. 1 is a view illustrating an endoscope apparatus that includes an endoscope and a sheath for an endoscope into which a flexible insertion portion of the endoscope is to be inserted and disposed;
Fig. 2 is a view illustrating the configuration of the sheath for an endoscope;
Fig. 3 is a view illustrating a procedure for mounting the sheath for an endoscope onto the endoscope;
Fig. 4 is a view illustrating a state in which the sheath for an endoscope is mounted on the endoscope;
Fig. 5 is a view illustrating an action of a stopper member, which is a view that illustrates a state in which a distal end face of a sheath body is disposed inside a region of a distal end portion, and a state in which the distal end portion is disposed inside the sheath body;
Fig. 6 is a view illustrating another configuration example of a second knob portion and a stopper member; and
Fig. 7 is a view illustrating the configuration of a sheath for an endoscope that includes two stopper members.

### Best Mode for Carrying Out the Invention

Preferred embodiments of the present invention are described hereunder with reference to the accompanying drawings.

Note that the respective components in the respective drawings used for the following description are displayed in a different contraction scale so as to be shown in a size that is recognizable in the drawings. Further, the present invention is not limited only to the quantity of components, the shapes of components, the ratios between the sizes of components, and the relative positional relationship between the respective components illustrated in the drawings.

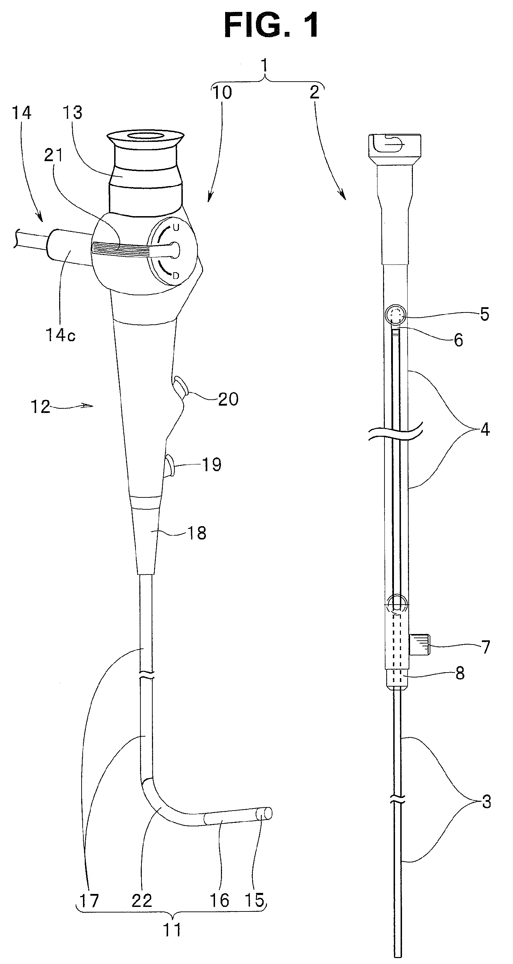

An endoscope apparatus 1 shown in Fig. 1 includes a sheath for an endoscope 2 and an endoscope 10.

The endoscope 10 is, for example, a nephroscope that is mainly constituted by an insertion portion 11, an operation portion 12 and an ocular portion 13. The ocular portion 13 is provided on a proximal end side of the operation portion 12.

Reference numeral 14 denotes a light guide. A connection connector 14c of the light guide 14 is detachably attached to a light guide connection port (not shown) that is provided at a side portion of the operation portion 12.

The insertion portion 11 includes, in order from the distal end side, a rigid distal end portion 15, a bending portion 16 that is configured to bend in the upward and downward directions, and a flexible tube portion 17 that is a tube body that is flexible which are connected in series.

A bend preventing portion 18 having a previously determined elastic force is provided on a proximal end side of the flexible tube portion 17. The bend preventing portion 18 is provided so as to cover the proximal end portion of the flexible tube portion 17 and prevent buckling of the flexible tube portion 17, and also is fixedly installed to maintain watertightness between the flexible tube portion 17 and the distal end side of the operation portion 12.

A water leakage detection pipe sleeve 19, a treatment instrument insertion port 20 and a bending operation lever 21 and the like are provided in the operation portion 12. The bending operation lever 21 is rotatable. The bending portion 16 bends in the two directions of upward and downward when bending wires (not shown) are pulled or slackened accompanying a rotational operation of the lever 21.

An observation window, an illuminating window and a treatment instrument opening, which are not shown in the drawings, are provided in a distal end face of the distal end portion 15 of the insertion portion 11. A bundle for illumination, a bundle for observation, a tube for a treatment instrument channel, an upward bending wire, a downward bending wire and the like, which are not shown in the drawings, are inserted through the inside of the insertion portion 11. The bundle for illumination is a component for transmitting illuminating light to the illuminating window. The bundle for observation is a component for transmitting an observation image that is to be observed through the observation window. The tube for a treatment instrument channel links the treatment instrument opening and the treatment instrument insertion port 20 to constitute a treatment instrument channel.

Note that reference numeral 22 denotes a passive bending portion that is configured to easily bend upon receiving an external force. The endoscope 10 is not limited to a nephroscope, and may be any endoscope having a flexible insertion portion in which the insertion portion 11 includes the bending portion 16 and the flexible tube portion 17.

The sheath for an endoscope 2 is mainly constituted by a sheath body 3 and an endoscope mounting portion 4. The sheath body 3 is a rigid metal member made of, for example, stainless steel. Reference numeral 5 denotes a knob portion. The knob portion 5 is fixedly installed in an integrated manner on the contact member 6. Reference numeral 7 denotes a fixing member that is a second knob portion in the present embodiment. The fixing member 7 is freely attachable to and detachable from a stopper member 8.

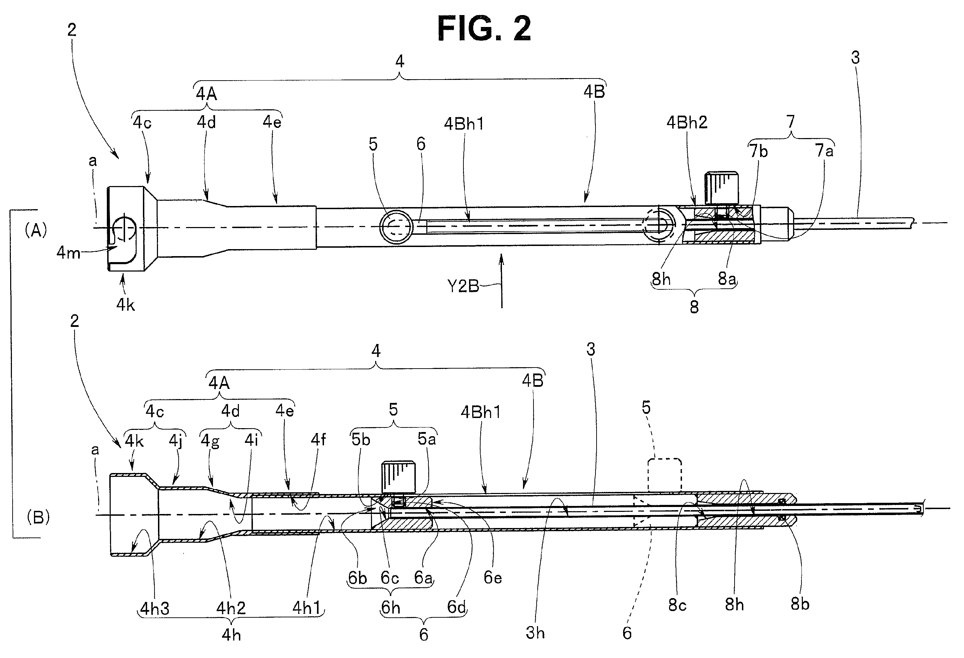

The configuration of the sheath for an endoscope 2 will now be described referring to (A) and (B) in Fig. 2. In Fig. 2, (A) is a front view that includes a partial cross-sectional view, and (B) is an explanatory view that includes a cross-sectional view when the sheath for an endoscope shown in (A) is seen from the direction of an arrow Y2B.

As shown in (A) in Fig. 2, the sheath body 3 is a first tubular member. The sheath body 3 is a so-called "pipe member" in which the diameter and inner diameter are constant, and which has a longitudinal axis direction through-hole that is a straight hole. The straight hole of the sheath body 3 is an insertion portion inserting hole 3h. The insertion portion 11 of the endoscope 10 can be disposed in an insertable/extractable manner in the insertion portion inserting hole 3h.

The endoscope mounting portion 4 is a second tubular member. The endoscope mounting portion 4 has a longitudinal axis direction through-hole 4h that has a stepped shape. In the present embodiment, the endoscope mounting portion 4 is constituted by integrally fixing together a main body pipe portion 4A and a sheath length adjustment pipe portion (hereunder, abbreviated as "sheath length adjustment portion") 4B by adhesion or bonding.

Note that the endoscope mounting portion 4 is not limited to a configuration in which a plurality of members are fixed in an integrated manner, and may be constituted by a single pipe member.

The main body pipe portion 4A is constituted by an operation portion arrangement portion 4c, a bend preventing arrangement portion 4d and a connecting portion 4e.

The connecting portion 4e includes a fitting hole 4f on an opening side. One end portion of the sheath length adjustment portion 4B that has a straight pipe shape is fitted and disposed inside the fitting hole 4f, and is integrally fixed therein by, for example, adhesion. An inner face of the fixed sheath length adjustment portion 4B and an inner face of the connecting portion 4e form substantially the same plane. The inner faces formed on the same plane serve as an inner face of a small-diameter straight hole 4h1 of the longitudinal axis direction through-hole 4h.

The bend preventing arrangement portion 4d is constituted by a tapered face portion 4g and a bend preventing contact portion 4i. The bend preventing contact portion 4i is an intersecting portion between an inner face of the tapered face portion 4g and an inner face of the connecting portion 4e. In a state in which the sheath for an endoscope 2 is arranged on the endoscope 10, the bend preventing contact portion 4i is pressingly disposed against the bend preventing portion 18.

Note that a taper angle of the tapered face portion 4g is previously set to be greater than a taper angle of the bend preventing portion 18. Further, the inner diameter of the inner face of the connecting portion 4e is set to a diametrical dimension at a previously determined intermediate portion of the bend preventing portion 18.

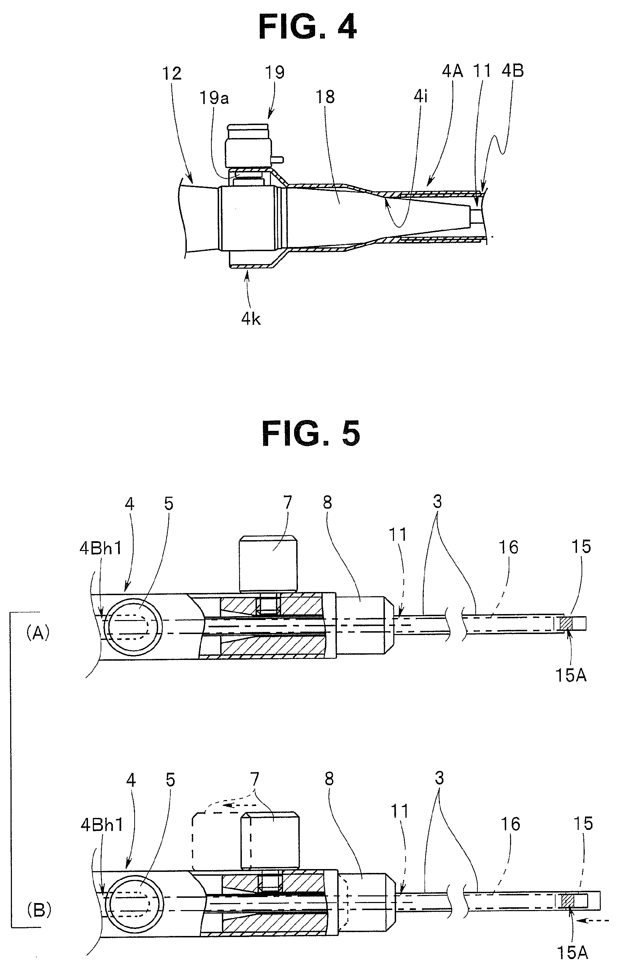

The operation portion arrangement portion 4c is constituted by a bend preventing covering portion 4j and a pipe sleeve arrangement portion 4k. The bend preventing covering portion 4j and one part of the tapered face portion 4g cover the circumference of the bend preventing portion 18. The pipe sleeve arrangement portion 4k covers the area around the vicinity of the water leakage detection pipe sleeve 19 of the operation portion 12. An engagement grove 4m that constitutes a locking portion is provided in the pipe sleeve arrangement portion 4k. A mirror image of the shape of the engagement grove 4m is an approximately "L" shape. The engagement grove 4m is engagingly disposed on a shaft portion (denoted by reference character 19a in Fig. 4) of the water leakage detection pipe sleeve 19.

The longitudinal axis direction through-hole 4h is mainly constituted by a small-diameter straight hole 4h1, a bend preventing covering hole 4h2 and an operation portion covering hole 4h3.

A through-hole of the sheath length adjustment portion 4B is the small-diameter straight hole 4h1. The contact member 6 and stopper member 8 are slidably disposed in the small-diameter straight hole 4h1.

As shown in (A) and (B) in Fig. 2, a movement range setting hole 4Bh1 and a positioning hole 4Bh2 are formed in the outer circumferential face of the sheath length adjustment portion 4B.

The movement range setting hole 4Bh1 is a long hole along a longitudinal axis a. The movement range setting hole 4Bh1 leads to the small-diameter straight hole 4h1 of the longitudinal axis direction through-hole 4h. The movement range setting hole 4Bh1 sets a movement range in the longitudinal axis direction of the contact member 6.

The positioning hole 4Bh2 is a through-hole that leads to the small-diameter straight hole 4h1. In the present embodiment, the positioning hole 4Bh2 is a long hole along the longitudinal axis a. The positioning hole 4Bh2 sets a movement range in the longitudinal axis direction of the stopper member 8.

One end of the movement range setting hole 4Bh1 is formed at a position that is separated by a previously determined distance from one end face of the endoscope mounting portion 4. The other end of the movement range setting hole 4Bh1 is formed at a position that is separated by a previously determined distance from the one end of the setting hole 4Bh1.

One end of the positioning hole 4Bh2 is formed at a position that is separated by a previously determined distance from the other end of the movement range setting hole 4Bh1. The other end of the positioning hole 4Bh2 is formed at a position that is separated by a previously determined distance from the one end of the positioning hole 4Bh2. The positioning hole 4Bh2 is provided at a position on the other end face side of the endoscope mounting portion 4.

The contact member 6 is a cylindrical shape and is slidably disposed in the small-diameter straight hole 4h1. The contact member 6 includes an axial direction through-hole 6h and a female thread portion 6d.

An axial direction hole portion 6a and an introducing hole portion are provided in the axial direction through-hole 6h.

The introducing hole portion 6b has an opening in one end face side that is a hole provided with a tapered face 6c. The tapered face 6c is a guide face for smoothly guiding the distal end portion 15 of the insertion portion 11 into the insertion portion inserting hole 3h of the sheath body 3. The diametrical dimension of the tapered face 6c is such that the diameter continuously decreases as the distance from the opening increases.

The axial direction hole portion 6a is a straight hole that has an opening in the other end face side. One end portion of the sheath body 3 is arranged in the axial direction hole portion 6a. A bottom face of the introducing hole portion 6b and a bottom face of the axial direction hole portion 6a communicate by means of a connection hole (not shown in the drawings). The diametrical dimension of the connection hole is set to approximately the same dimension as the diametrical dimension of the insertion portion inserting hole 3h.

The one end portion of the sheath body 3 is integrally fixed by, for example, adhesion to the axial direction hole portion 6a. As a result, the sheath body 3 is provided in a protruding condition from the other end face side of the contact member 6. In the fixedly installed state, the insertion portion inserting hole 3h of the sheath body 3 and the introducing hole portion 6b are linked by a communicating hole.

The female thread portion 6d is made by forming a female thread in an inner face of a concave portion provided in a side face of the contact member 6. An axis of the concave portion is orthogonal to an axis of the axial direction through-hole 6h.

A male thread portion 5a that is provided on the knob portion 5 is threadedly disposed in the female thread portion 6d. The male thread portion 5a is inserted through the inside of the movement range setting hole 4Bh1 formed in the endoscope mounting portion 4 and is threadedly engaged with the female thread portion 6d. The knob portion 5 is disposed integrally in the contact member 6 in a state in which the endoscope mounting portion 4 is interposed therebetween.

The contact member 6 is fixed integrally to the endoscope mounting portion 4 by adjusting the threadedly engaged state by pressingly disposing a pressing face 5b of the knob portion 5 against the external surface of the endoscope mounting portion 4. In contrast thereto, the contact member 6 is arranged so as to freely advance and retreat in the direction of the longitudinal axis a of the endoscope mounting portion 4 inside the small-diameter straight hole 4h1 by adjusting the threadedly engaged state so as to provide a gap between the pressing face 5b of the knob portion 5 and the external surface of the endoscope mounting portion 4.

The distal end face of the sheath body 3 comes closest to the distal end face of the endoscope mounting portion 4 when the knob portion 5 is moved inside the movement range setting hole 4Bh1 to cause the knob portion 5 to abut against one end thereof. On the other hand, the distal end face of the sheath body 3 is furthest from the distal end face of the endoscope mounting portion 4 at a time that the knob portion 5 is caused to abut against the other end.

The stopper member 8 is a cylindrical shape and is slidably disposed in the small-diameter straight hole 4h1. The stopper member 8 includes a holding hole 8h that is an axial direction through-hole and a female thread portion 8a.

The holding hole 8h is a through-hole that holds the sheath body 3 in a manner such that the sheath body 3 is capable of advancing and retreating. The sheath body 3 that protrudes from the other end face side of the contact member 6 is inserted through the holding hole 8h.

One end face of the stopper member 8 is a contact portion that a contact face 6e that is the other end face of the contact member 6 contacts against.

The female thread portion 8a is made by forming a female thread in an inner face of a concave portion provided in a side face of the stopper member 8. An axis of the concave portion is orthogonal to an axis of the holding hole 8h. An opening of the female thread portion 8a opens in a direction orthogonal to the axis of the holding hole 8h.

A male thread portion 7a that is provided on a second knob portion 7 that is a positioning and fixing member is threadedly disposed in the female thread portion 8a. The male thread portion 7a is inserted through the inside of the positioning hole 4Bh2 that is a long hole formed in the endoscope mounting portion 4, and is threadedly engaged with the female thread portion 8a. The second knob portion 7 is disposed integrally in the stopper member 8 in a state in which the endoscope mounting member 4 is interposed therebetween.

The stopper member 8 is fixed integrally to the endoscope mounting portion 4 by adjusting the threadedly engaged state by pressingly disposing a pressing face 7b of the second knob portion 7 against the external surface of the endoscope mounting portion 4. In contrast thereto, the stopper member 8 is arranged so as to freely advance and retreat in the direction of the longitudinal axis a of the endoscope mounting portion 4 inside the small-diameter straight hole 8h1 by adjusting the threadedly engaged state so as to provide a gap between the pressing face 7b of the second knob portion 7 and the external surface of the endoscope mounting portion 4.

A position that the contact face 6e of the contact member 6 contacts against is changed by moving the stopper member 8 inside the positioning hole 4Bh2. That is, a distance between the distal end face of the endoscope mounting portion 4 and the distal end face of the sheath body 3 is adjusted by adjusting the position at which the stopper member 8 is disposed.

In the present embodiment, an O-shaped ring 8b that is an urging member is arranged inside the holding hole 8h on the other end face side of the stopper member 8. The O-shaped ring 8b is a sliding resistance member, and imparts a desired sliding resistance to the sheath body 3. Accordingly, the O-shaped ring 8b closely contacts the external surface of the sheath body 3 that is inserted through the inside of the holding hole 8h, in a state in which the O-shaped ring 8b is deformed by a previously determined amount.

In a state in which sliding resistance is imparted to the sheath body 3 from the O-shaped ring 8b, the sheath body 3 is prevented from moving under its own weight with respect to the endoscope mounting portion 4, even if the state is one in which the contact member 6 disposed in the small-diameter straight hole 4h1 can advance/retreat in the direction of the longitudinal axis a of the endoscope mounting portion 4.

Reference character 8c denotes a tapered face. The tapered face 8c is provided on one end face side of the holding hole 8h. Similarly to the tapered face 6c, the tapered face 8c is constituted so that the diametrical dimension continuously decreases as the distance from the opening increases.

The tapered face 8c of the holding hole 8h is a guide face for smoothly guiding an end portion of the sheath body 3 into the holding hole 8h.

The action of the sheath for an endoscope 2 configured as described above will now be described.

The endoscope 10 for performing an observation or the like inside a kidney, and the sheath for an endoscope 2 are prepared. A physician or a medical worker mounts the sheath for an endoscope 2 on the endoscope 10 in advance.

At such time, the medical worker (hereunder, referred to as "operator") moves the stopper member 8 to the most distal end side as shown in (A) in Fig. 2, and thereafter pressingly disposes the pressing face 7b of the second knob portion 7 against the external surface of the endoscope mounting portion 4. Further, as shown by a dashed line in (B) in Fig. 2, the operator causes the contact face 6e of the contact member 6 to contact against the contact portion of the stopper member 8, and thereafter pressingly disposes the pressing face 5b of the knob portion 5 against the external surface of the endoscope mounting portion 4.

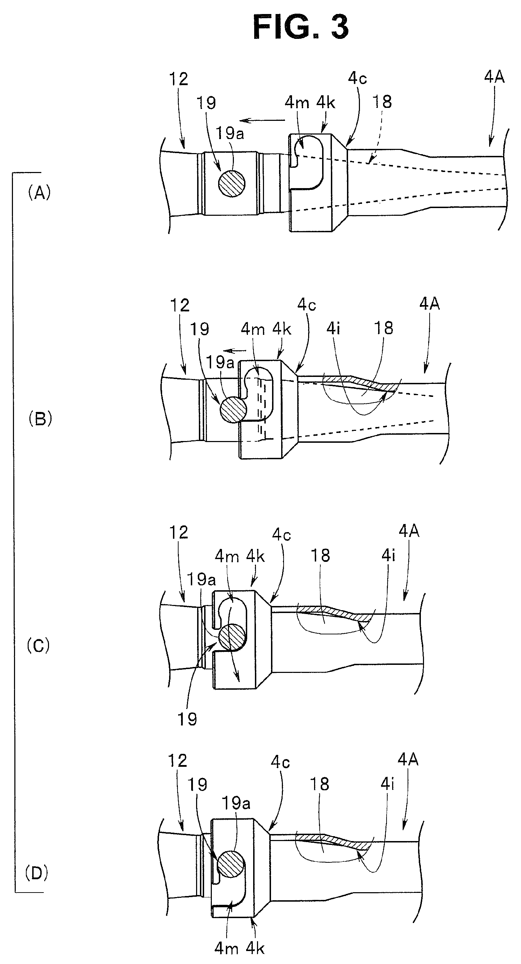

Thereafter, the operator inserts the distal end portion 15 of the insertion portion 11 of the endoscope 10 towards the inside of the insertion portion inserting hole 3h of the sheath body 3 through the operation portion covering hole 4h3 that has an opening on the proximal end side of the sheath for an endoscope 2. Thereupon, as shown in (A) in Fig. 3, the operation portion arrangement portion 4c of the main body pipe portion 4A included in the sheath for an endoscope 2 approaches the water leakage detection pipe sleeve 19 that is provided in the operation portion 12.

In this case, as shown in (B) in Fig. 3, the operator brings the main body pipe portion 4A close to the water leakage detection pipe sleeve 19 while performing a positional adjustment so that the opening of the engagement grove 4m is engagingly disposed at the water leakage detection pipe sleeve 19. Thereupon, the bend preventing contact portion 4i of the main body pipe portion 4A contacts against the bend preventing portion 18 of the endoscope 10.

Next, the operator moves the main body pipe portion 4A further against the elastic force of the bend preventing portion 18 and, as shown in (C) of Fig. 3, moves the main body pipe portion 4A by pushing in the main body pipe portion 4A so that the water leakage detection pipe sleeve 19 is disposed inside the engagement grove 4m. At this time, the bend preventing portion 18 gradually undergoes elastic deformation accompanying movement of the main body pipe portion 4A.

Next, the operator subjects the main body pipe portion 4A to a twisting operation to, as shown in (D) of Fig. 3, dispose the water leakage detection pipe sleeve 19 inside a deep part of the groove which is a part that is separated from the opening of the engagement grove 4m. Thus, as shown in (D) in Fig. 3 and in Fig. 4, the engagement grove 4m is engagingly disposed around a shaft portion 19a of the water leakage detection pipe sleeve 19 and mounting of the sheath for an endoscope 2 to the endoscope 10 is completed.

At this time, the engagement grove 4m is in a state of being urged to the distal end side of the insertion portion 11 at all times by the elastic force of the bend preventing portion 18. As a result, the engagement grove 4m is reliably and stably fixed on the shaft portion 19a.

Note that, in the present embodiment, the bend preventing covering portion 4j and the pipe sleeve arrangement portion 4k are provided in the operation portion arrangement portion 4c, and the engagement grove 4m is provided in the pipe sleeve arrangement portion 4k. Further, the engagement grove 4m is engagingly disposed on the shaft portion 19a of the water leakage detection pipe sleeve 19 to perform mounting of the sheath for an endoscope 2 onto the endoscope 10.

However, mounting of the sheath for an endoscope 2 to the endoscope 10 is not limited to engagement of the engagement grove 4m onto the shaft portion 19a of the water leakage detection pipe sleeve 19, and as long as the engagement grove 4m is engaged with a convex portion that protrudes from the operation portion 12, mounting of the sheath for an endoscope 2 may be performed to a pipe sleeve other than the water leakage detection pipe sleeve 19 or to a pipe sleeve or the like constituting the treatment instrument insertion port 20.

For example, in a case where the engagement grove is engaged with a shaft portion of a pipe sleeve of the treatment instrument insertion port 20, a configuration may be adopted in which the bend preventing covering portion 4j and an insertion pipe sleeve arrangement portion are provided on the operation portion arrangement portion 4c, and an engagement grove is provided in the insertion pipe sleeve arrangement portion to enable mounting of the sheath for an endoscope 2 to the endoscope 10.

Next, the operator checks whether or not the distal end face of the sheath body 3 included in the sheath for an endoscope 2 is disposed at a previously determined position on the outer circumferential face of the distal end portion 15 of the insertion portion 11.

In this case, as shown in (A) in Fig. 5, if the distal end face of the sheath body 3 is disposed inside a previously determined region 15A on the outer circumferential face of the distal end portion 15, the operator ends the mounting operation. In contrast, as shown in (B) in Fig. 5, if the distal end portion 15 is disposed inside the sheath body 3, the operator moves the stopper member 8 rearward in the direction of the bending portion 16 of the insertion portion 11.

At this time, the operator adjusts the threadedly engaged state of the knob portion 5 so as to provide a gap between the pressing face 5b and the external surface of the endoscope mounting portion 4, and adjusts the threadedly engaged state of the second knob portion 7 so as to provide a gap between the pressing face 7b and the external surface of the endoscope mounting portion 4. Thereafter, the operator moves the second knob portion 7 in the arrow direction that is indicated by a dashed line to gradually retract the stopper member 8. The operator then adjusts the disposing positions so that the distal end face of the sheath body 3 is positioned inside the previously determined region 15A on the outer circumferential face of the distal end portion 15.

After adjusting the disposing positions, the operator adjusts the threadedly engaged state of the knob portion 5 and the threadedly engaged state of the second knob portion 7, respectively, and pressingly disposes the pressing face 5b against the external surface of the endoscope mounting portion 4 and also pressingly disposes the pressing face 7b against the external surface of the endoscope mounting portion 4 to thereby complete the mounting work.

Finally, the operator adjusts the threadedly engaged state of the knob portion 5 so as to enter a state in which a gap is provided between the pressing face 5b and the external surface of the endoscope mounting portion 4. The operator then moves the contact member 6 in a retreating direction and checks whether or not the bending portion 16 and the passive bending portion 22 are completely exposed. Further, in a case where the bending portion 16 and the passive bending portion 22 are completely exposed before the knob portion 5 arrives at one end of the movement range setting hole 4Bh1, the operator attaches, for example, a notification seal for notifying the physician of the relevant position to the external surface of the sheath length adjustment portion 4B.

Subsequently, after checking whether or not the bending portion 16 is completely exposed, the operator again places the contact face 6e of the contact member 6 in a state in which the contact face 6e contacts against the contact portion of the stopper member 8. Thereafter, the operator pressingly disposes the pressing face 5b of the knob portion 5 against the external surface of the endoscope mounting portion 4 to thereby complete the mounting work.

Subsequently, to perform an observation inside a kidney, the physician inserts the insertion portion 11 of the endoscope 10 on which the sheath body 3 of the sheath for an endoscope 2 is mounted into the urinary duct, and introduces the insertion portion 11 towards the inside of the kidney. At this time, because the flexible insertion portion 11 is being covered and made rigid by the sheath body 3, the distal end portion 15 of the insertion portion 11 is smoothly introduced into the kidney.

Upon confirming through the ocular portion 13 that the distal end portion 15 is introduced into the kidney, the physician adjusts the threadedly engaged state of the knob portion 5 to enter a state in which a gap is provided between the pressing face 5b and the external surface of the endoscope mounting portion 4. At this time, the physician moves the contact member 6 in a retreating direction to enter a state in which the bending portion 16 and the passive bending portion 22 are completely exposed.

Thereafter, the physician appropriately operates the bending operation lever 21 to cause the bending portion 16 to perform a bending operation and carries out observation or treatment inside the kidney. After completing the observation or treatment, the physician extracts the insertion portion 11 of the endoscope 10 on which the sheath for an endoscope 2 is mounted from inside the urinary duct to thereby end the endoscopic procedure.

Thus, the sheath body 3 that covers the flexible insertion portion 11 of the endoscope 10 is configured to be capable of advancing/retreating by a previously determined distance with respect to the endoscope mounting portion 4 constituting the sheath for an endoscope 2.

According to this configuration, by covering the flexible insertion portion 11 with the sheath body 3, the insertion portion 11 can be smoothly introduced into a kidney via the urinary duct, for example.

Further, after being introduced into the kidney, the physician moves the sheath body 3 by a previously determined distance with respect to the endoscope mounting portion 4 to expose the bending portion 16 that is provided in the insertion portion 11. By this means, the physician can cause the bending portion 16 to perform a bending operation and can observe the inside of the kidney over a wide range.

Further, in addition to having a configuration such that the sheath body 3 is capable of advancing/retreating by a previously determined distance in the longitudinal axis direction of the endoscope mounting portion 4, the stopper member 8 for changing the disposing position of the distal end of the sheath body 3 is configured to move forward/rearward by a previously determined distance in the longitudinal axis direction of the endoscope mounting portion 4.

According to this configuration, even in a case where there is a difference in length due to an individual difference in the insertion portion 11 of the endoscope 10 that is combined with the sheath for an endoscope 2, by adjusting the disposing position of the stopper member 8 it is possible to reliably dispose the distal end face of the sheath body 3 constituting the sheath for an endoscope 2 inside the previously determined region 15A on the outer circumferential face of the distal end portion 15 of the insertion portion 11.

Further, the O-shaped ring 8b that imparts sliding resistance to the sheath body 3 is provided inside the holding hole 8h of the stopper member 8 and closely contacts the external surface of the sheath body 3 that is inserted through the inside of the holding hole 8h. By this means, even in a state in which the contact member 6 can freely advance/retreat in the direction of the longitudinal axis a of the endoscope mounting portion 4, the sheath body 3 can be prevented from moving under its own weight with respect to the endoscope mounting portion 4.

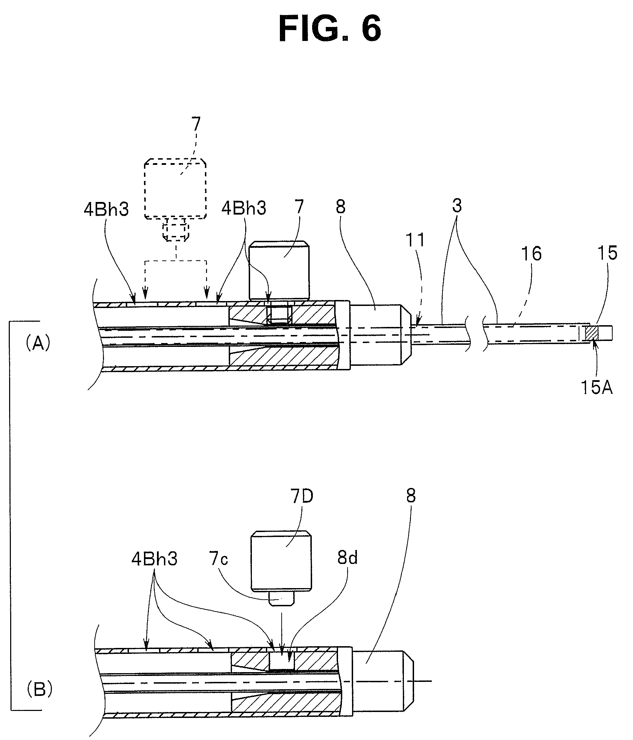

Note that, in the above-described embodiment, a configuration is adopted in which the disposing position of the stopper member 8 can be adjusted by moving the second knob portion 7 along the positioning hole 4Bh2 that is a long hole. However, instead of the positioning hole 4Bh2, a configuration may also be adopted in which a plurality of positioning holes 4Bh3 constituted by a plurality of, for example, round holes that are through-holes that lead to the small-diameter straight hole 4h1 are arranged in the longitudinal axis direction as shown in (A) of Fig. 6.

According to this configuration, when changing the position of the stopper member 8, as indicated by dashed lines, the second knob portion 7 is taken out once from the stopper member 8, the operator then moves the stopper member 8, and thereafter threadedly engages the second knob portion 7 with the stopper member 8 again. As a result, adjustment of the disposing position of the stopper member 8 can be performed.

In the above-described embodiment, the fixing member is configured as the second knob portion having a male thread portion. However, the fixing member is not limited to a knob portion having a male thread portion. For example, as shown in (B) in Fig. 6, the fixing member may be a locking pin 7D in which a pin portion 7c protrudes from a distal end of a knob or the like. In a configuration that uses the locking pin 7D instead of the second knob portion, an engagement hole 8d is provided instead of the female thread 8a in the stopper member 8.

According to this configuration, when changing the position of the stopper member 8, the pin portion 7c of the locking pin 7D is engagedly inserted into the engagement hole 8d through a desired positioning hole 4Bh3. As a result, adjustment of the disposing position of the stopper member 8 can be performed.

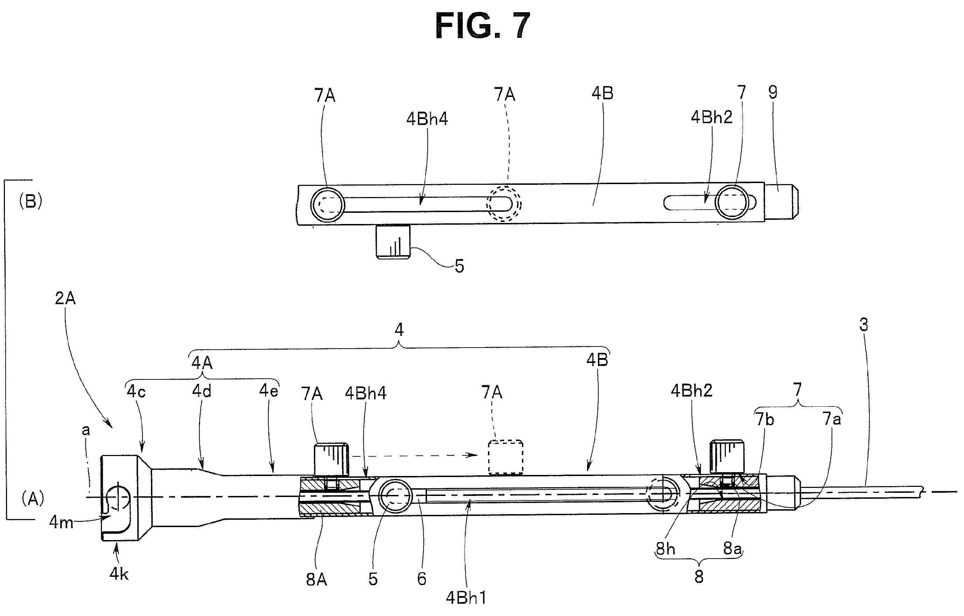

In addition, in the above-described embodiment, the distance by which the distal end face of the sheath body 3 is separated from the distal end face of the endoscope mounting portion 4 is adjusted by adjusting the disposing position of the stopper member 8 provided on the distal end side of the sheath length adjustment portion 4B. However, as shown in (A) and (B) in Fig. 7, a configuration may also be adopted in which a second stopper member 8A is provided in addition to the stopper member 8 to enable adjustment of the distance by which the distal end face of the sheath body 3 is separated from the distal end face of the endoscope mounting portion 4.

Note that the second stopper member 8A has the same configuration as that of the stopper member 8. Further, in a sheath for an endoscope 2A in which the second stopper member 8A is provided, a second positioning hole 4Bh4 that is a long hole along the longitudinal axis a that leads to the small-diameter straight hole 4h1 is provided on the proximal end side of the sheath length adjustment portion 4B in addition to the positioning hole 4Bh2. Note that the positioning hole 4Bh4 sets the movement range of the second stopper member 8A in the longitudinal axis direction.

According to this configuration, after the operator performs adjustment of the disposing position by gradually retracting the above-described stopper member 8 so that the distal end face of the sheath body 3 is positioned inside the previously determined region 15A on the outer circumferential face of the distal end portion 15, if the bending portion 16 and the passive bending portion 22 are completely exposed before the knob portion 5 reaches one end of the movement range setting hole 4Bh1, the operator disposes and fixes the second stopper member 8A at the relevant position instead of attaching a notification seal to the external surface of the sheath length adjustment portion 4B.

As a result, when the physician causes the contact member 6 to move rearward after confirming through the ocular portion 13 that the distal end portion 15 is introduced into the kidney, without viewing a notification seal the physician can place the bending portion 16 and the passive bending portion 22 in a completely exposed state by bringing the contact face of the proximal end side of the contact member 6 into contact with the contact portion on the distal end side of the second stopper member 8A.

Note that in a case where, for example, there is a request from the physician to dispose the distal end face of the sheath body 3 at a portion that is partway along the passive bending portion 22 to perform fine adjustment of the bending function, the operator disposes and fixes the second stopper member 8A in accordance with the request of the physician.

Thus, the second stopper member 8A is provided in addition to the stopper member 8 on the sheath for an endoscope 2A. By this means, if there is an individual difference in the length or the like of the bending portion 16 or the length or the like of the passive bending portion 22 in addition to a difference in length that is caused by an individual difference of the insertion portion 11 of the endoscope 10, by adjusting the disposing position of the second stopper member 8A, an adjustment that corresponds to a request of the physician can be easily performed, such as reliably exposing the bending portion 16 and the passive bending portion 22.

Note that, in the above described configuration, the positioning hole 4Bh4 that differs with the positioning hole 4Bh2 is provided in the main body pipe portion 4A. However, a configuration may also be adopted in which the positioning hole 4Bh2 and the positioning hole 4Bh4 are configured as a dual-purpose positioning hole that is constituted by a single long hole that is formed in a straight line.

Further, the positioning hole 4Bh4 may be constituted by a plurality of through-holes similarly to the positioning hole 4Bh2.

It should be understood that the present invention is not limited only to the above described embodiments, and various modifications can be made without departing from the spirit and scope of the invention.

The present application claims priority from Japanese Patent Application No. 2013-265324 filed in Japan on December 24, 2013, the contents of which are hereby incorporated by reference in their entirety into the description, the claims and the drawings of this application.

## Claims

1. A sheath for an endoscope, comprising:
a first tubular member having a longitudinal axis direction through-hole in which an insertion portion of an endoscope that has a distal end portion, a bending portion and a flexible tube portion can be disposed in an insertable/extractable manner;
a stopper member that has a holding hole that holds the first tubular member so that the first tubular member is capable of advancing and retreating, and a concave portion forming an opening in a direction perpendicular to an axis of the holding hole;
a contact member that is integrally fixed to one end portion side of the first tubular member which is held so as to be capable of advancing and retreating inside the holding hole of the stopper member and which protrudes from one end face side of the stopper member, and that has a contact face that contacts against one end face of the stopper member;
a second tubular member that includes a longitudinal axis direction through-hole in which the contact member and the stopper member can be slidably disposed, a locking portion that is provided on a one end-face opening side of the longitudinal axis direction through-hole and that can be locked to an operation portion of the endoscope, a movement range setting hole that is a long hole leading to the longitudinal axis direction through-hole and that sets a longitudinal axis direction movement range of the contact member with which the first tubular member is integrated, and a positioning hole that is a through-hole leading to the longitudinal axis direction through-hole and that is formed so as to be alignable with respect to the concave portion of the stopper member that is provided on the other end-face opening side of the longitudinal axis direction through-hole;
a positioning and fixing member that is disposed in the concave portion of the stopper member through the positioning hole that the second tubular member includes, and that positions and fixes the stopper member with respect to the second tubular member; and
a knob portion that is integrally fixed to the contact member through the movement range setting hole that the second tubular member includes.

2. The sheath for an endoscope according to claim 1, wherein the positioning hole is an elongated long hole along a longitudinal axis direction of the second tubular member or is a plurality of through-holes provided in the longitudinal axis direction.

3. The sheath for an endoscope according to claim 2, wherein the concave portion of the stopper member is a threaded portion having a female thread, and the positioning and fixing member has a male thread that is threadedly disposed in the female thread of the threaded portion through the long hole or a through-hole.

4. The sheath for an endoscope according to claim 2, wherein the concave portion of the stopper member is an engagement hole, and the positioning and fixing member has a pin portion that is engagedly inserted into the engagement hole through the through-hole.

5. The sheath for an endoscope according to claim 1, further comprising an urging member that closely contacts an external surface of the first tubular member inside the holding hole provided in the stopper member and imparts sliding resistance to the first tubular member.

6. The sheath for an endoscope according to claim 1, wherein:
in a configuration in which a first stopper member for adjusting a disposing position of a distal end face of the first tubular member on the distal end portion of the endoscope, and a second stopper member for adjusting the disposing position of the distal end face of the first tubular member to a position at which the bending portion of the endoscope is in a desired exposed state are provided in the longitudinal axis direction through-hole of the second tubular member,
in the second tubular member, a positioning hole is provided for disposing a positioning and fixing member for positioning and fixing each of the stopper members with respect to the second tubular member in a concave portion of the stopper member.

7. The sheath for an endoscope according to claim 6, comprising, in the second tubular member, a first positioning hole for disposing a first fixing member in the concave portion of the first stopper member, and a second positioning hole for disposing a second fixing member in the concave portion of the second stopper member.

8. The sheath for an endoscope according to claim 7, wherein a dual-purpose positioning hole that is used as both the first positioning hole and the second positioning hole is provided in the second tubular member.

9. The sheath for an endoscope according to any one of claims 1 to 8, wherein a sliding resistance member is provided between the holding hole of the stopper member and the first tubular member.

10. The sheath for an endoscope according to claim 9, wherein the sliding resistance member is an O-shaped ring.
